# EUROPÄISCHE PATENTANMELDUNG

(11) **EP 2 388 027 A1**
(43) Veröffentlichungstag der Anmeldung: **23.11.2011**
(21) Anmeldenummer: 10075213.8
(22) Anmeldetag: 20.05.2010
(51) Int. Cl.: A61M 1/10

(54) **Pumpensystem mit einem Förderbetrieb und einem Sonderbetrieb**

(71) Anmelder: Berlin Heart GmbH, 12247 Berlin (DE)
(72) Erfinder: Arndt, Andreas, 12555 Berlin (DE); Donath, Johannes, 10829 Berlin (DE)
(74) Vertreter: Pfenning, Meinig & Partner GbR

(57) **Zusammenfassung**

Die Erfindung bezieht sich auf eine Blutpumpe (5), die insbesondere mittels eines Rotors angetrieben ist, und erlaubt im Rahmen eines neuartigen Pumpensystems und eines Verfahrens zu dessen Betrieb durch den Wechsel zwischen einem Förderbetrieb und einem Sonderbetrieb die zeitweilige Änderung der Strömungsbedingungen und die Umkehr eines Gesamtvolumenstroms der Pumpe, wodurch die Neigung zur Koagulation von Blut im Bereich der Pumpe verringert oder verhindert wird. Ein Weg zu einer geeigneten Ansteuerung der Pumpe zur Erreichung dieses Effekts ist die zeitweilige Absenkung der Pumpendrehzahl in beträchtlichem Maß.

## Beschreibung

Die Erfindung liegt auf dem Gebiet des Maschinenbaus und der Mikromechanik mit Anwendungen auf dem medizinischen Sektor, insbesondere bei der Gestaltung von Pumpen kleiner Bauart zur Anwendung bei der Herzunterstützung in Blutkreislaufsystemen von Säugetieren.

Vielfältige Flüssigkeitspumpen kleiner Bauart sind bekannt geworden, die zur Herzunterstützung bei ungenügender Funktion speziell des menschlichen Herzens vorübergehend oder dauerhaft eingesetzt werden können. Derartige Pumpen können invasiv oder nichtinvasiv in den Blutkreislauf eingeschaltet werden.

Ein Beispiel ist dabei die Einführung einer derartigen Pumpe über ein Blutgefäß bis zu einer oder in eine Herzkammer über einen Herzkatheter, wobei die entsprechende Pumpe üblicherweise einen komprimierten Zustand für den Transport und einen potentiell expandierten Zustand für den Betrieb nach dem Transport aufweist.

Ein weiteres Beispiel ist eine implantierbare Blutpumpe, die über Kanülen an Blutgefäße und Herz angeschlossen wird und eine Unterstützung des Herzens, beispielsweise der linken Herzkammer, dauerhaft bewirken kann.

Oft handelt es sich bei derartigen Pumpen um Rotorpumpen, die einen antreibbaren Rotor mit Förderelementen zur axialen oder zentrifugalen Förderung des Bluts aufweisen. Verschleißfrei kann ein derartiger Rotor beispielsweise magnetisch gelagert sein, um lange Standzeiten zu erreichen.

Ebenso wie im normalen Blutkreislauf des Menschen teilweise ungewollte Koagulationen des Blutes stattfinden, die sich bis zu Thrombosen entwickeln können, kann dies auch mit einer eingebrachten Pumpe nicht ausgeschlossen werden.

Die Erfindung setzt sich daher zum Ziel, derartige Koagulationseffekte des Bluts im Umfeld einer Pumpe zu vermeiden und/oder zu verringern.

Diese Aufgabe wird mit den Merkmalen der Erfindung gemäß Patentanspruch 1 gelöst.

Die Erfindung sieht dazu bei einem Pumpensystem eine Blutpumpe vor, die ein von Blut durchströmbares Gehäuse und einen Antrieb mit einem Förderelement aufweist sowie eine Steuerungseinrichtung zur Steuerung der Blutpumpe, wobei die Steuerungseinrichtung die Pumpe abwechselnd im Förderbetrieb und in einem Sonderbetrieb betreibt, wobei im Sonderbetrieb wenigstens ein Antriebsparameter der Pumpe so weit geändert wird, dass an wenigstens einer Stelle entlang des Strömungswegs durch die Pumpe der Gesamtvolumenstrom wenigstens zeitweise umgekehrt wird.

Üblicherweise ist eine derartige Blutpumpe mit einem rotatorischen Antrieb aufgebaut, beispielsweise als Axialpumpe mit einem Impeller/Propeller oder als Zentrifugalpumpe. Es sind aber auch andere Pumpenformen, beispielsweise für den pulsatilen Betrieb oder allgemein mit mechanischen Antriebselementen, in der Form von volumenveränderbaren Verdrängungselementen zur Förderung eines Fluids denkbar. Zudem sind alle anderen Prinzipien, darunter Membranpumpen oder auch Pumpen mit einem Antrieb mittels Einwirkung von elektrischen oder magnetischen Feldern zur Anwendung der Erfindung denkbar.

Für die Erfindung erweist es sich als vorteilhaft, dass von Zeit zu Zeit eine Sonderbetriebsphase zwischen Förderbetriebsphasen eingelegt wird, wobei die Sonderbetriebsphase eine Änderung wenigstens eines Antriebsparameters wenigstens in dem Maße vorsieht, dass wenigstens an einer Stelle entlang des Strömungsweges durch die Pumpe der Gesamtvolumenstrom wenigstens zeitweise umgekehrt wird.

Als änderbarer Antriebsparameter kommen dabei bei Pumpen mit rotierenden Elementen vorzugsweise die Antriebs-/Rotationsdrehzahl in Frage.

Die Erfindung ist beispielsweise auf Pumpen mit rotatorisch antreibbaren Förderelementen wie Propellern derart anwendbar, dass die Drehzahl verändert oder der Anstellwinkel eines Förderelements/Propellers geändert wird. Es kann auch der Expansionsgrad, beispielsweise Aufklappwinkel von Förderelementen geändert werden, wenn dies gezielt möglich ist. Bei Pumpen, die sich zur Förderung des Fluids eines volumenveränderlichen Elements bedienen, kann die Amplitude der Volumenänderung bzw. die Frequenz der Volumenänderung entsprechend verändert werden. Maßgebend für die Wirkung der Erfindung ist insbesondere, dass sich an vielen Stellen der Strömung, besonders dort wo Koagulationen stattfinden können, die Strömungsverhältnisse zumindest vorübergehend ändern oder sogar umkehren. Es sind in diesem Zusammenhang so genannte Totwassergebiete bekannt, in denen sehr geringe Strömungsgeschwindigkeiten vorherrschen und somit Koagulationen begünstigt sein können. Zudem können derartige Koagulationen auch an Strömungshindernissen generell begünstigt sein. Die Erfindung kann vorteilhaft durch die Änderung der Antriebsparameter gerade in solchen Bereichen Änderungen der Strömungsverhältnisse bewirken. Dabei wird sich üblicherweise der Gesamtvolumenstrom durch die Pumpe zeitweise umkehren. Unter der Voraussetzung einer inkompressiblen geförderten Flüssigkeit kann jedoch trotzdem der Gesamtvolumenstrom an verschiedenen Stellen entlang des Strömungswegs unterschiedlich sein, wenn kurzzeitige Gehäusevolumenänderungen in Betracht gezogen werden. Insbesondere bei Pumpengehäusen mit flexiblen Membranen können solche Gehäusevolumenänderungen bei Änderungen der Strömungsverhältnisse auftreten, wenn diese auch im Normalbetrieb von untergeordneter Bedeutung sind.

In der Aorta ist dies durch die physiologisch wirksame sogenannte Windkesselfunktion erreicht, die im normalen physiologischen Ablauf während der Höchstdruckphase der Systole durch ein Nachgeben der Gefäßwände eine Speicherung eines Blut-Teilvolumens bewirkt, das bei Absinken des Drucks durch das Zusammenziehen der Gefäßwände nachgetrieben wird. Dadurch wird die Blutströmung vergleichmäßigt. Im vorliegenden Fall eines Sonderbetriebs kann der Effekt bis zu einem Rücktreiben des Blutes entgegen der Hauptströmungsrichtung führen.

Zudem kann durch eine Sonderbetriebsphase eine Spülung des Ventrikels erreicht werden, und es kann eine gelegentliche Öffnung der Aortenklappe erfolgen.

Eine vorteilhafte Ausgestaltung der Erfindung sieht vor, dass die Blutpumpe eine Einlassöffnung und eine Auslassöffnung aufweist, wobei eine Reduktion der Förderleistung der Pumpe während des Sonderbetriebs derart bemessen ist, dass an wenigstens einer Stelle zwischen der Einlassöffnung und der Auslassöffnung der Flächenmittelwert des Axialgeschwindigkeitsprofils gemittelt über den Strömungsquerschnitt wenigstens einmal vorübergehend sein Vorzeichen wechselt.

Dabei wird unter dem Axialgeschwindigkeitsprofil das Profil der Axialgeschwindigkeiten von infinitesimalen Volumenelementen über einen Querschnitt des Strömungsweges verstanden. Das Integral über diesen Strömungsquerschnitt bezüglich der Axialgeschwindigkeitsverteilung ergibt somit die Gesamtstromstärke. Ändert sich der Mittelwert des Axialgeschwindigkeitsprofils, so geht damit eine Änderung des Integrals und somit der Gesamtstromstärke des Flüssigkeitsstroms einher.

Bereits Änderungen im Axialgeschwindigkeitsprofil können sich gemäß der Erfindung vorteilhaft auf die Verhinderung von Koagulationen auswirken, da dies die Verlagerung von Wirbeln innerhalb eines Strömungsquerschnitts bedeutet. Als besonders vorteilhaft hat sich jedoch die wenigstens zeitweise Umkehrung der Gesamtströmung an einem Punkt entlang des Strömungsweges herausgestellt.

Dabei hat sich als besonders vorteilhaft für die Erfindung herausgestellt, wenn der Gesamtvolumenstrom an einer Einlassöffnung und/oder einer Auslassöffnung des Pumpengehäuses wenigstens einmal seine Richtung zeitweise umkehrt. Es kann dabei durchaus auch vorgesehen sein, dass der Gesamtvolumenstrom während eines Sonderbetriebs mehrmals eine dem Förderbetrieb entgegengesetzte Richtung und zwischendurch kurzzeitig eine dem Förderbetrieb entsprechende Richtung einnimmt. Dies erzeugt aufeinander folgende Richtungsänderungen der Strömung, wodurch ein nichtstationärer Strömungsverlauf mit chaotischer Wanderung der Verwirbelungen erreicht wird, so dass besonders wahrscheinlich sämtliche Volumina innerhalb des Pumpengehäuses mit wechselnden Strömungsbedingungen beaufschlagt werden können.

Besonders vorteilhaft ist, wenn während eines Sonderbetriebs an mehreren Stellen, insbesondere an allen Stellen des Strömungswegs durch die Pumpe der Gesamtvolumenstrom wenigstens einmal zeitweise umgekehrt ist.

Es hat sich weiterhin als vorteilhaft herausgestellt, wenn das Zeitintegral des Förderstroms über eine gesamte Sonderbetriebsphase negativ ist.

Dies bedeutet, dass die Pumpe tatsächlich zeitweise ein gewisses Volumen von der Auslassöffnung zur Einlassöffnung befördert, das erst nach Wiederaufnahme des Förderbetriebs durch die Pumpe hindurch bewegt wird.

Die Erfindung bezieht sich weiterhin auf ein Pumpensystem mit einer Blutpumpe, die ein von Blut durchströmbares Gehäuse und einen Antrieb mit einem Förderelement aufweist sowie mit einer Steuerungseinrichtung zur Steuerung der Blutpumpe. Zudem sieht die Erfindung vor, dass das Förderelement der Pumpe als Rotor ausgebildet ist und dass in der Steuerungseinrichtung wenigstens ein erstes Drehzahlniveau des Rotors für den Förderbetrieb und wenigstens ein Sonderbetriebsdrehzahlniveau für einen Sonderbetrieb vorgesehen ist, wobei das wenigstens eine Sonderbetriebsdrehzahlniveau eine niedrigere Drehzahl des Rotors vorsieht als der Förderbetrieb.

Vorteilhaft ist im Sonderbetrieb die Sonderbetriebsdrehzahl so weit abgesenkt, dass die oben beschriebene Umkehr des Gesamtvolumenstroms wenigstens zeitweise und wenigstens an bestimmten Stellen des Strömungsweges erreicht wird.

Es kann zudem vorgesehen sein, dass über eine Zeitspanne während einer Sonderbetriebsphase ein konstantes Sonderbetriebsdrehzahlniveau, insbesondere das niedrigste Sonderbetriebsdrehzahlniveau, vorgesehen ist.

In diesem Fall kann die Drehzahl zu Beginn des Sonderbetriebs über eine lineare Rampe verringert, dann auf ein Sonderbetriebsrehzahlniveau konstant gehalten und danach über eine weitere Rampe wieder bis zum Förderbetrieb gesteuert werden.

Es ist auch eine sägezahnförmige Verlaufssteuerung der Drehzahl möglich, so dass die minimale Drehzahl im Sonderbetrieb nur für einen Augenblick erreicht wird.

Die Erfindung kann sich zudem auf ein Pumpensystem mit einer Blutpumpe beziehen, die ein von Blut durchströmbares Gehäuse und einen Antrieb mit einem Förderelement aufweist sowie eine Steuerungseinrichtung zur Steuerung der Blutpumpe, wobei das Förderelement der Pumpe als Rotor ausgebildet ist, wobei vorgesehen ist, dass die Steuerungseinrichtung ein erstes Drehzahlniveau des Rotors für den Förderbetrieb und wenigstens ein Sonderbetriebsdrehzahlniveau für einen Sonderbetrieb vorsieht, und wobei die Drehzahl während eines Sonderbetriebs wenigstens zeitweise wenigstens 2000 U/min, insbesondere 2500 U/min, weiter insbesondere 3000 U/min oder 4000 U/min unterhalb der Drehzahl im Normalbetrieb liegt.

Bei üblichen Drehzahlen von Blutpumpen mit Rotoren, die beispielsweise bei 7500 U/min oder auch bei 10000 U/min liegen können, wird mit den genannten Maßnahmen der Drehzahlverminderung bereits in vielen Fällen eine Umkehr der Förderrichtung des Blutes im Sonderbetrieb erreicht.

Die Erfindung bezieht sich außer auf ein Pumpensystem der oben beschriebenen Art auch auf ein Verfahren zum Betrieb einer Blutpumpe, die in den Blutkreislauf eines Patienten eingeführt ist, wobei die Pumpe abwechselnd im Förderbetrieb und im Sonderbetrieb angesteuert wird, wobei der Sonderbetrieb wenigstens zeitweise eine Umkehrung des Gesamtvolumenstroms an wenigstens einer Stelle entlang des Strömungsweges zwischen einer Einlassöffnung der Pumpe und der nächsten Querschnittsverengung des Strömungsweges hinter der Auslassöffnung der Pumpe insbesondere zwischen der Einlassöffnung der Pumpe und der Auslassöffnung der Pumpe bewirkt.

Entsprechende Sonderbetriebsphasen können vorteilhaft zwischen 0,5 Sekunden und 1,5 Sekunden lang sein und beispielsweise wenigstens zweimal pro Minute, einmal pro Minute oder nur alle fünf bis zehn Minuten zwischen den Förderbetriebsphasen eingeschoben werden.

Gemäß des erfindungsgemäßen Betriebsverfahrens kann zudem vorgesehen sein, dass während einer Sonderbetriebsphase die Drehzahl der Pumpe bis zu einer Sonderbetriebsdrehzahl abgesenkt und danach wieder angehoben wird.

Zur Erreichung des gewünschten Effekts auf die Strömung können auch andere Parameter der Pumpe, wie ein Anstellwinkel von Förderschaufeln/Rotorblättern, geändert werden. Wird gleichzeitig die Strömungsrichtung und/oder der Druck an geeigneten Stellen des Strömungsweges erfasst, so kann ein entsprechender Parameter auch geregelt werden. Üblicherweise genügt jedoch eine Steuerung, besonders einfach eine Steuerung der Drehzahl.

Dazu kann erfindungsgemäß vorgesehen sein, dass das wenigstens ein Sonderbetriebsdrehzahlniveau um wenigstens 25%, insbesondere 40%, weiter insbesondere 50% oder 60%, oder um wenigstens 2000 U/min oder 3000 U/Min unterhalb der Drehzahl des Förderbetriebs liegt. Dabei können vorteilhaft 0,1 bis 2 Sonderbetriebsphasen, insbesondere zwischen 1 und 2 Sonderbetriebsphasen, pro Minute eingelegt werden.

Im Folgenden wird die Erfindung anhand eines Ausführungsbeispiels in einer Zeichnung gezeigt und nachfolgend beschrieben.

### Dabei zeigt

- Fig. 1: eine schematische Übersicht über eine Blutpumpe, die mittels eines Katheters in eine Herzkammer wenigstens teilweise eingeführt ist,
- Fig. 2: eine schematische Übersicht über eine Steuerungseinrichtung sowie ein Diagramm, das den Betrieb einer Pumpe beschreibt,
- Fig. 3: schematisch den Aufbau einer Pumpe in dreidimensionaler Ansicht,
- Fig. 4: schematisch den Aufbau einer Rotationspumpe als Zentrifugalpumpe in dreidimensionaler Ansicht,
- Fig. 5: schematisch den Aufbau einer Pumpe, die mit einem steuerbaren Verdrängungselement arbeitet,
- Fig. 6: eine Pumpe in dreidimensionaler Ansicht, die, wie eine Pumpe aus Fig. 5 mit einem volumenveränderlichen Expansionselement arbeitet,
- Fig. 7: schematisch den Strömungsverlauf in einem Pumpengehäuse,
- Fig. 8: schematisch den Zeitverlauf der Drehzahl einer Pumpe bzw. eines Pumpenrotors,
- Fig. 9: schematisch eine Ansicht eines Pumpengehäuses mit einem Rotor und der entsprechenden Fluidströmung,
- Fig. 10: eine weitere schematische Ansicht der Strömung durch ein Pumpengehäuse,
- Fig. 11: in einem Diagramm den zeitlichen Verlauf der Drehzahl einer Pumpe,
- Fig. 12: eine weitere Verlaufsform der Pumpendrehzahl in einem Diagramm.

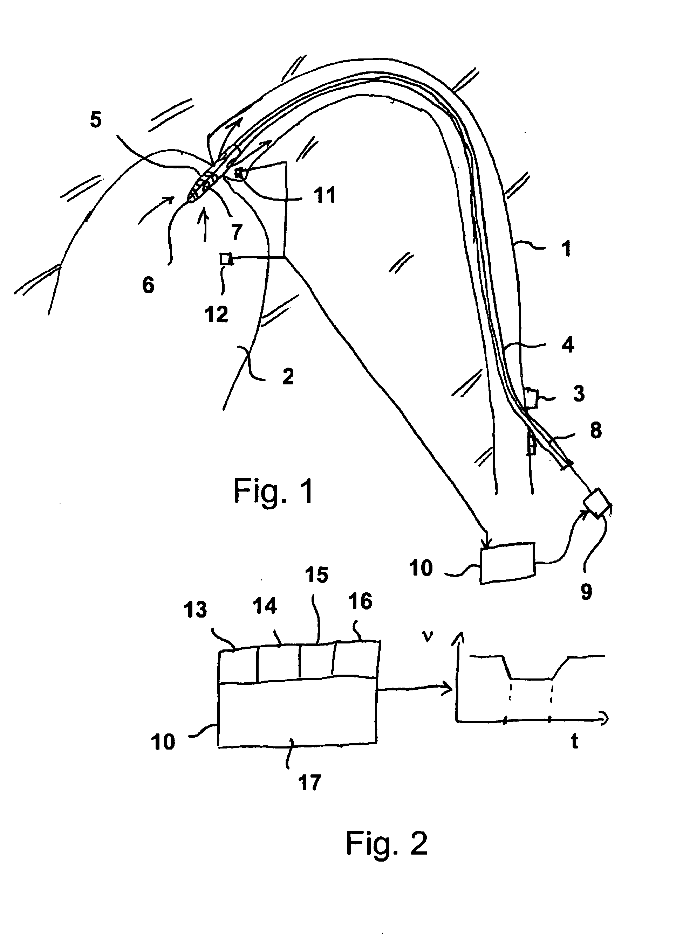

Fig. 1 zeigt schematisch ein Blutgefäß 1, das in einem Ventrikel 2 einer Herzkammer mündet, wobei durch das Blutgefäß 1 über eine Schleuse 3 ein Katheter 4 eingeführt ist.

Der Katheter 4 reicht bis fast in das Ventrikel 2 hinein, wobei am Ende des Katheters eine Blutpumpe 5 angeordnet ist, die teilweise in das Ventrikel hineinragt.

Die Blutpumpe 5 ist in der Lage, über eine Ansaugöffnung 6 Blut anzusaugen und dieses über Ausströmöffnungen innerhalb des Blutgefäßes 1 wieder auszustoßen und somit die Herzfunktion wenigstens zu unterstützen oder teilweise zu ersetzen.

Die Pumpe 5 weist in ihrem Inneren innerhalb eines Gehäuses einen Rotor 7 auf, der mittels einer antreibbaren Welle 8 rotatorisch in einem Drehzahlbereich von beispielsweise 7000 bis 10000 U/min antreibbar ist. Die Welle 8 ragt dabei proximal aus dem Katheter 4 heraus und ist mit einem Antriebsmotor 9 verbunden.

Der Antriebsmotor 9 ist mittels einer Steuerungseinrichtung 10 ansteuerbar oder regelbar. Dabei können beispielsweise bestimmte Drehzahlen oder zeitabhängige Drehzahlverläufe vorgegeben sein.

Die Steuereinrichtung 10 kann optional mit einem oder mehreren Drucksensoren 11, 12 verbunden sein, die beispielsweise in dem Blutgefäß 1, dem Pumpengehäuse oder dem Ventrikel 2 positioniert werden können, um dort den aktuellen Druck zu messen. Diese Druckmessung kann als Eingangsgröße beispielsweise für eine Regelung innerhalb der Steuerungseinrichtung 10 verwendet werden. Es ist jedoch im Normalfall eine reine Steuerung der Pumpendrehzahlen ausreichend. Eine Messung der Geschwindigkeitsverteilung des Blutstroms kann auch über eine Doppler-Spektroskopieeinrichtung erfolgen, deren Analyseergebnisse ebenfalls als Eingangsgrößen für eine Regelung dienen können.

Fig. 2 zeigt eine schematische Darstellung der Steuerungseinrichtung, die über einen ersten Speicher 13 zur Speicherung einer Pumpendrehzahl im Förderbetrieb verfügt sowie über einen zweiten Speicher 14 zur Speicherung einer Pumpendrehzahl während des Sonderbetriebs. Zudem weist die Steuerungseinrichtung 10 einen dritten Speicher 15 zur Speicherung der zwischen zwei Sonderbetriebsphasen liegenden Förderbetriebszeit auf sowie einen vierten Speicher 16 zur Speicherung der Dauer einer Sonderbetriebsphase bzw. der Steilheit der Rampen bei der Änderung der Drehzahl zum Übergang zwischen dem Förderbetrieb und dem Sonderbetrieb.

Die Steuerungseinrichtung weist zudem einen Funktionsteil 17 auf, der mittels einer Zeitsteuerung unter Verwendung der gespeicherten Daten die jeweils aktuelle Zieldrehzahl an den Motor 9 ausgibt.

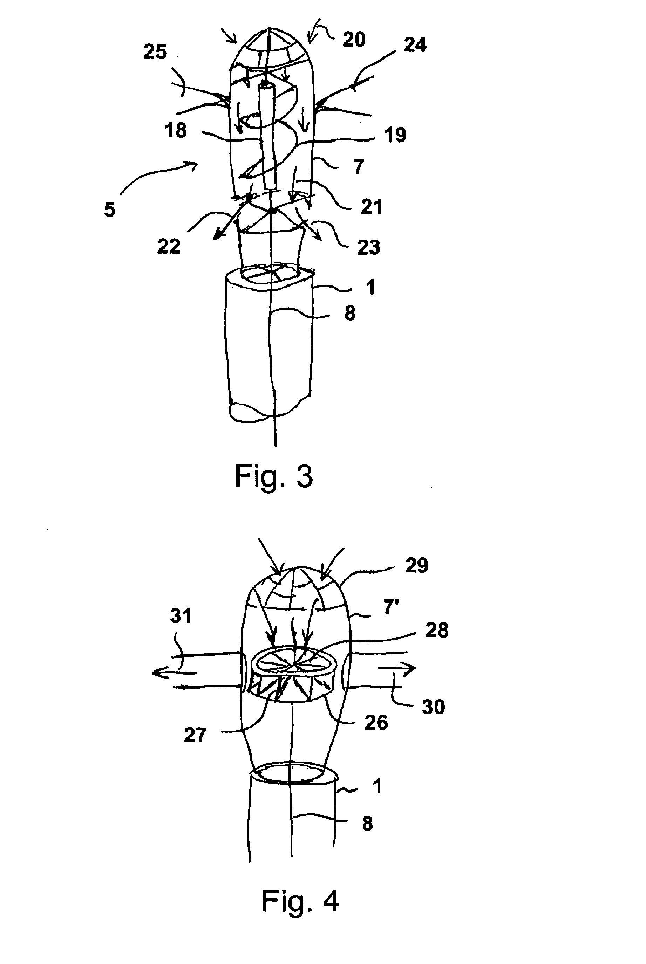

Fig. 3 zeigt einen typischen Pumpenaufbau einer Pumpe 5 zur Anwendung der Erfindung mit einem Gehäuse 7, wobei in dem Gehäuse eine Nabe 18 drehbar gelagert ist, die mit einer Antriebswelle 8 verbunden ist.

Die Nabe trägt dabei wenigstens ein Förderelement 19 in Form eines Propellers bzw. einer helixartigen Struktur, die bei Rotation einen Axialschub auf eine Flüssigkeit, dargestellt durch Strömungspfeile 20, 21 erzeugt. Die angesaugte Flüssigkeit wird über Ausströmöffnungen hinter der Pumpe ausgestoßen, was durch die Strömungspfeile 22, 23 angedeutet ist.

Die Pumpe 5 ist am Ende eines Katheters 1 an diesem befestigt.

Schematisch sind Herzklappen 24, 25 angedeutet, die zur Abdichtung der Pumpe beim Durchgang/Übergang zwischen dem Blutgefäß 1 und dem Ventrikel 2 dienen.

Die Fig. 4 zeigt eine weitere Pumpe zur Anwendung der Erfindung, die ebenfalls einen Rotor aufweist wie die in Fig. 3 gezeigte Pumpe, jedoch nach dem Zentrifugalprinzip arbeitet. Hierzu ist in dem Pumpengehäuse 7' ein Rotor 26 mit Schaufelflächen 27 drehbar gelagert. Der Rotor 26 ist mittels der Antriebswelle 8 mit hoher Geschwindigkeit antreibbar, so dass über eine Stirnfläche 28 des Rotors 26 Flüssigkeit vom Kopfende 29 der Pumpe angesaugt und zentrifugal über den Umfang des Pumpengehäuses 7', wie durch die Pfeile 30, 31 angedeutet, ausgestoßen werden kann. Am Umfang der Pumpe kann ein Kanal angeordnet sein, der das nach außen gepresste Blut auffängt und einer Auslassöffnung zuführt.

Auch eine solche Pumpe ist üblicherweise am Ende eines Katheters 1 angeordnet. Ihre Drehzahl kann gemäß dem erfindungsgemäßen Verfahren ebenso wie die der Pumpe aus Fig. 3 gesteuert werden.

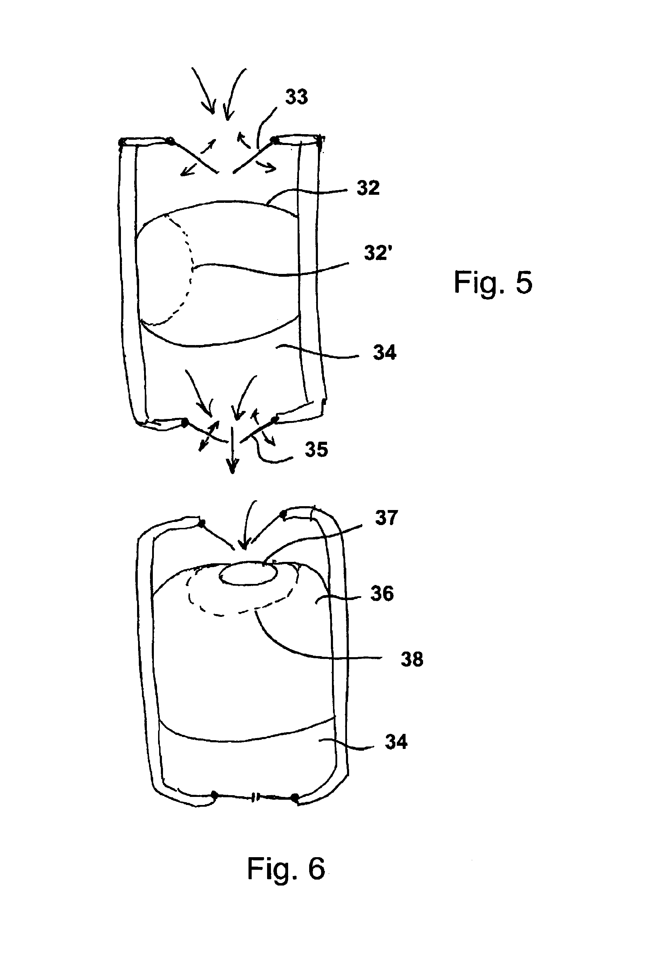

Fig. 5 zeigt eine Pumpe, die ohne rotatorische Antriebselemente auskommt und lediglich ein bezüglich seines Volumens steuerbares Antriebselement 32 in Form eines füllbaren Kissens aufweist. Das Kissen 32 ist im vollen Expansionszustand mit durchgezogenen Linien dargestellt, während die gestrichelte Linie das Kissen im komprimierten Zustand 32' darstellt.

Wird das Kissen komprimiert, so wird über die Eingangsöffnung durch entsprechendes Klappen des Einwegventils 33 Flüssigkeit in den Pumpeninnenraum 34 angesaugt. Das Ventil 33 ist ein Einwegventil, das das Herausströmen von Flüssigkeit aus dem Pumpeninnenraum verhindert. Dagegen ist das Auslassventil 35 als Einwegventil nur für den Auslass einer Flüssigkeit aus dem Pumpeninnenraum 34 konzipiert. Daher sperrt bei einer erneuten Expansion des Kissens 32 das Einlassventil 33 und die verdrängte Flüssigkeit kann aus dem Pumpeninnenraum 34 durch das Auslassventil 35 ausgelassen werden. Eine derartige Pumpe erlaubt einen pulsatilen Betrieb mit einem periodisch schwankenden Förderstrom.

In der Fig. 6 ist eine ähnliche Pumpe wie in Fig. 5 dargestellt, wobei das Kissen 36 in seiner Mitte eine Öffnung 37 aufweist, die in expandiertem Zustand des Kissens 36, als durchgezogene Linie dargestellt, relativ klein ist, jedoch das Durchströmen einer Flüssigkeit erlaubt.

Im komprimierten Zustand des Kissens 36 ist die Öffnung, wie anhand der gestrichelten Linie 38 dargestellt, stark vergrößert, so dass in dem Pumpeninnenraum 34 Flüssigkeit angesaugt wird. Die Funktion des Einlass- und Auslassventils an den Enden des Pumpeninnenraums 34 ist identisch zu der Darstellung in Fig. 5, so dass durch periodisches Komprimieren und Expandieren des Kissens 36 ein schwankender Förderstrom erzeugt wird, der bezüglich seiner Geschwindigkeitsamplitude und der Frequenz der Volumenänderungen steuerbar ist.

Aus der Beschreibung der Fign. 3, 4, 5 und 6 wird deutlich, dass innerhalb der jeweiligen Pumpen, unabhängig vom Antriebsprinzip, von dem getriebenen Fluidstrom verschiedene Pumpenteile beströmt oder durchströmt werden, was im Detail an manchen Stellen zu einer gegenüber dem Durchschnittswert gesteigerten oder verringerten Strömungsgeschwindigkeit infolge von Wirbelbildung oder ähnlichen Effekten führt. Dies kann insbesondere an einem Rotor oder einem Antriebskissen der Fall sein oder auch bei Stützrädern, die die entsprechenden Pumpengehäuse stützen sowie an anderen Stützstrukturen von Pumpengehäusen, deren Außenhaut beispielsweise als biegeschlaffe Membran aufgebaut werden kann.

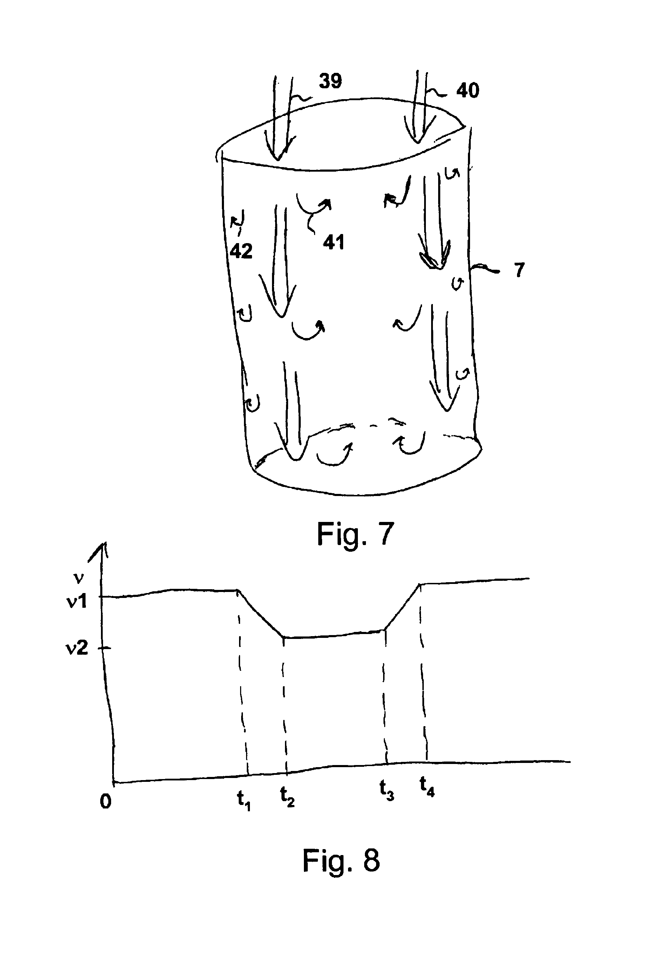

Die Fig. 7 zeigt schematisch und beispielhaft einen Strömungsverlauf durch ein Pumpengehäuse 7, das idealistisch zylindrisch angenommen ist. In diesem Fall sind zwei Hauptströmungspfeile 39, 40 dargestellt, von denen sich kleinere Wirbel 41, 42 zu allen Seiten hin lösen. Dies führt zu einer nicht ganz homogenen Strömungsverteilung innerhalb des Pumpenquerschnitts. Es müssen dabei nicht notwendig tatsächlich rückströmende Wirbel erzeugt werden, es kann auch an einer Stelle des Pumpenquerschnitts ein inhomogenes Axialgeschwindigkeitsprofil ohne Rückströmungskomponenten erzeugt werden.

Fig. 8 zeigt ein Diagramm mit einer im zeitlichen Verlauf gemäß der Erfindung gesteuerten Drehzahl υ einer Blutpumpe bzw. eines Pumpenrotors einer solchen Pumpe auf der vertikalen Achse. Auf der horizontalen Achse ist dabei der Zeitverlauf dargestellt.

Zum Zeitpunkt 0 ist dabei die Drehzahl υ₁ des normalen Förderbetriebs, typischerweise 7500 U, angenommen, die bis zum Zeitpunkt t₁ konstant gehalten wird. Zwischen dem Zeitpunkt t₁ und t₂ fällt die Drehzahl im Laufe der Zeit linear von υ₁ auf υ₂ ab, um dann zwischen t₂ und t₃ auf der Drehzahl υ₂ konstant gehalten zu werden. υ₂ stellt damit die minimale Sonderbetriebsdrehzahl dar.

Eine solche Drehzahl kann beispielsweise bei 3000 oder 3500 U/min liegen.

Nach dem Zeitpunkt t₃ wird die Drehzahl über eine lineare Rampe zwischen t₃ und t₄ wieder auf die Förderbetriebsdrehzahl υ₁ gesteigert. Danach kann der Förderbetrieb beispielsweise für eine halbe Minute oder eine Minute konstanter Drehzahl weitergefahren werden, bevor die nächste Sonderbetriebsphase erreicht wird.

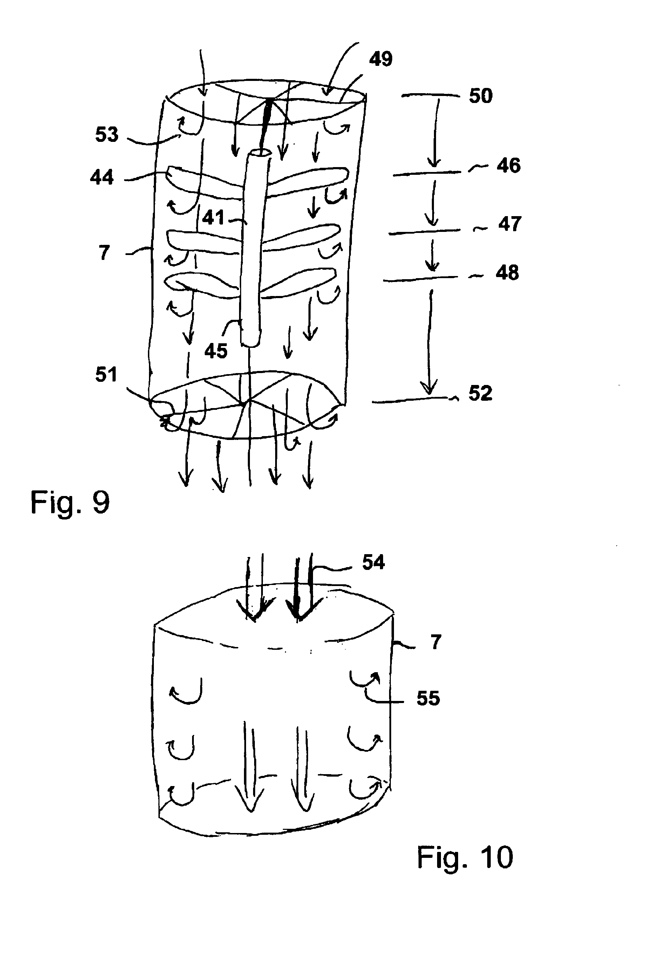

Die Fig. 9 zeigt ein Pumpengehäuse 7 mit einem Rotor 43, der Propellerblätter 44 aufweist, wobei beispielsweise drei Propellerblätterpaare in drei verschiedenen Abschnitten einer Nabe 45 vorgesehen sind. Die axialen Positionen dieser Propellerblätterpaare sind schematisch auf der rechten Seite der Darstellung mit 46, 47, 48 bezeichnet.

Zudem ist schematisch ein Stützrad 49 mit Speichen auf einer axialen Position 50 sowie ein Stützrad 51 auf einer axialen Position 52 dargestellt. Die Stützräder 49, 51 dienen dazu, die Gehäusemembran des Gehäuses 7, die grundsätzlich biegeschlaff sein kann, zu stützen und entsprechende axiale Einlassöffnungen sowie Auslassöffnungen des Gehäuses offen zu halten.

Die Strömung, die bei dem ersten Stützrad 49 in das Pumpengehäuse 7 eintritt, kann an verschiedenen Stellen entlang des Strömungsweges Wirbel ausbilden, wie dies an dem Wirbel 53 beispielhaft gezeigt ist, der an dem ersten Stützrad 49 im Bereich einer Speiche entsteht.

Wirbel können zudem an den Propellerpaaren entstehen, die in den axialen Höhen 46, 47, 48 entlang des Pumpengehäuses positioniert sind. Letztlich können Wirbel auch an dem zweiten Stützrad 51, beispielsweise im Bereich von dessen Speichen, entstehen. Grundsätzlich können Wirbel selbstverständlich auch im Bereich der Gehäusewand des Pumpengehäuses und der Nabe 45 entstehen.

Es ist evident, dass durch eine Änderung der Strömungsgeschwindigkeit innerhalb der Pumpe die Verwirbelungen in ihrem Ausmaß geändert werden und in ihrer räumlichen Verteilung wandern können.

Somit ist es durch die Erfindung möglich, Strömungsverhältnisse derart zu ändern, dass die Koagulation von Blut durch zeitweilige Durchströmung von sonst schwach durchströmten Gebieten verhindert oder verlangsamt wird.

Hierzu kann es ausreichen, an einem der Punkte entlang des Strömungsweges, also an den Stellen 46, 47, 48, 50, 52, eine signifikante Änderung der Strömungsverteilung bzw. eine Umkehr der Summenströmung zu erreichen; es kann jedoch auch sinnvoll sein, dies an mehreren Stellen entlang der Strömung gleichzeitig oder entlang des gesamten Strömungsweges zu bewirken. Dieser Zustand einer Strömungsumkehr kann kurzfristig erreicht werden, um danach wieder die im Förderbetrieb übliche Förderrichtung anzunehmen; während einer Sonderbetriebsphase kann ein solcher Wechsel der Strömungsrichtung auch mehrfach vorgesehen sein.

Es ist jedoch auch denkbar, während einer Phase des Sonderbetriebs für eine Zeitlang die umgekehrte Strömungsrichtung aufrechtzuerhalten und damit eine Nettoförderung eines Fluidvolumens entgegen der üblichen Förderrichtung zu erzeugen.

Dies geschieht in Folge einer gesunkenen Förderleistung bzw. Drehzahl der Pumpe und der äußeren Druckbedingungen außerhalb der Pumpe, die der Strömungsrichtung während des normalen Förderbetriebs als Widerstand/Gegendruck entgegenwirken. Die sogenannte Windkesselfunktion der Aorta bewirkt durch die Elastizität der Gefäßwände, die im Bereich des höchsten Drucks nachgeben, eine Speicherung eines Blut-Teilvolumens und bei Absenken des Drucks ein Nach- bzw. Zurückpumpen. Der Überdruck am Pumpenauslass kann damit eine Rückströmung durch eine Druckdifferenz zum Pumpeneinlass bewirken. Diese Verhältnisse sind beim Einsatz in einem Herzen auch von der Phase des Herzschlages abhängig, so dass die Einleitung einer Sonderbetriebsphase auch auf den Herzschlag abgestimmt werden kann. Insbesondere kann die Sonderbetriebsphase derart gesteuert werden, dass zeitweilig kein Blut aus dem Herzventrikel angesaugt wird und dieses sich vollständig füllen kann.

Auch eine Rückströmung aus dem Blutgefäß in das Herzventrikel kann vorteilhaft sein, um den erfindungsgemäßen Effekt zu erreichen.

Das Herzventrikel kann durch eine Sonderbetriebsphase ebenfalls gespült und eine gelegentliche Öffnung der Aortenklappe erreicht werden.

Die Fig. 10 zeigt schematisch ein ideal zylindrisch angenommenes Pumpengehäuse 7, in das zentrisch entlang der Zylinderachse eine starke Strömung, dargestellt durch die Pfeile 54, eingeleitet wird. Es ist gezeigt, dass beträchtliche Verwirbelungen 55 im Bereich der Gehäusewand entstehen. Wird die Strömung 54 durch eine Verringerung des Pumpenschubs, beispielsweise durch Verringerung der Drehzahl eines Rotors oder Veränderung eines Anstellwinkels von Rotorpropellern, verringert/verlangsamt, so ist denkbar, dass die Verwirbelungen 55 im Zuge der Erzeugung einer laminaren Strömung verringert werden oder verschwinden. Dies würde eine Änderung der Strömungsbedingungen im Bereich der Pumpengehäusewand und damit ein Freispülen dieser Bereiche bewirken.

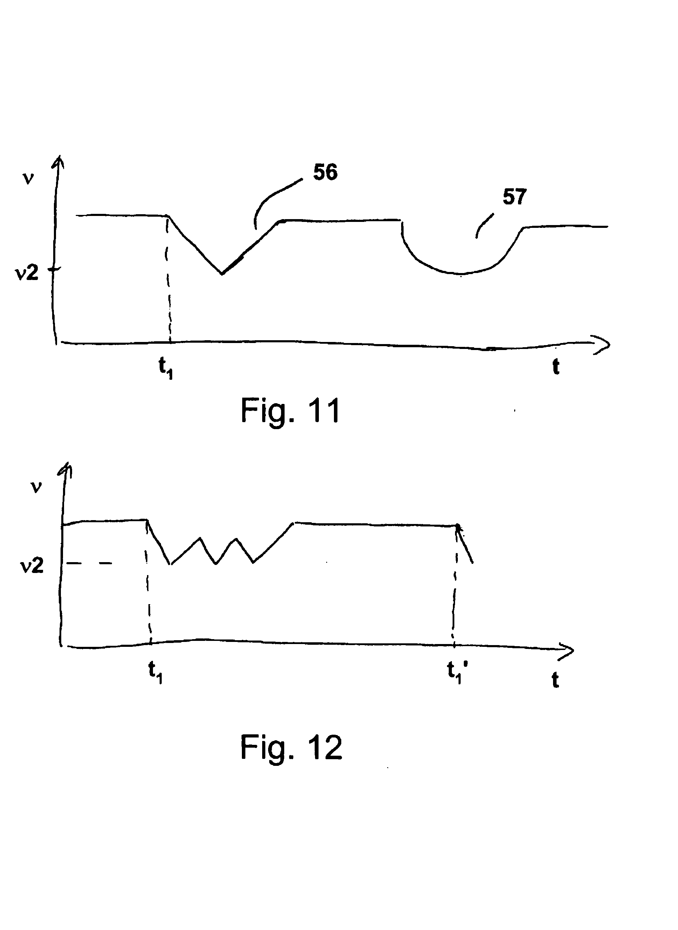

Die Fig. 11 zeigt exemplarisch zwei mögliche Verläufe von Drehzahlen eines Rotors einer Blutpumpe, vertikal gegenüber der Zeit t auf der horizontalen Achse aufgetragen.

Der erste Verlauf, mit 56 bezeichnet, stellt einen sägezahnförmigen Drehzahlverlauf dar, in dessen Rahmen, beginnend zu einem Zeitpunkt t₁, die Drehzahl υ von einer Förderbetriebsdrehzahl linear auf eine minimale Sonderbetriebsdrehzahl υ₂ abgesenkt und nach Erreichen dieser Sonderbetriebsdrehzahl υ₂ augenblicklich linear wieder bis zum Erreichen der Förderbetriebsdrehzahl gesteigert wird.

In dem Bereich 57 ist ein abgerundeter Verlauf der Drehzahl mit einer vorübergehenden Absenkung auf υ₂ dargestellt.

Das Diagramm aus Fig. 12 zeigt eine weitere Struktur, bei der, beginnend zu einer Zeit t₁, die Drehzahl υ von der Drehzahl des Förderbetriebs auf eine minimale Sonderbetriebsdrehzahl υ₂ abgesenkt und danach unmittelbar ausgehend von dieser minimalen Sonderbetriebsdrehzahl ein Stück weit angehoben wird. Dies geschieht mehrmals während der Sonderbetriebsphase, so dass die Gesamtströmungsrichtung des Blutstroms in dieser Phase mehrfach umgekehrt werden kann. Dies kann für die Ausbildung einer chaotischen Strömungsgeschwindigkeitsverteilung in der Sonderbetriebsphase vorteilhaft sein, um an jedem Punkt des Strömungsquerschnittes eine signifikante Änderung der Strömungsgeschwindigkeit vorübergehend zu erreichen, bevor wieder der Förderbetrieb aufgenommen wird.

Insgesamt erlaubt das erfindungsgemäße Pumpensystem und der Betrieb eines derartigen Systems gemäß der Erfindung eine Änderung der Strömungsbedingungen, die, von Zeit zu Zeit, insbesondere periodisch, wiederholt, auf Dauer die Ausbildung von Blutkoagulationen verhindert oder wenigstens verlangsamt.

## Patentansprüche

1. Pumpensystem mit einer Blutpumpe (5), die ein von Blut durchströmbares Gehäuse (7) und einen Antrieb mit einem Förderelement (19, 26, 27, 32, 36) aufweist, sowie mit einer Steuerungseinrichtung (10) zur Steuerung der Blutpumpe, **dadurch gekennzeichnet, dass** die Steuerungseinrichtung die Blutpumpe abwechselnd im Förderbetrieb und in einem Sonderbetrieb betreibt, wobei während des Sonderbetriebs wenigstens ein Antriebsparameter der Pumpe so weit geändert wird, dass an wenigstens einer Stelle (46, 47, 48, 50, 52) entlang des Strömungswegs durch die Pumpe der Gesamtvolumenstrom wenigstens zeitweise umgekehrt wird.

2. Pumpensystem nach Anspruch 1 mit einer Blutpumpe, die eine Einlassöffnung und eine Auslassöffnung aufweist, wobei eine Reduktion der Förderleistung der Pumpe während des Sonderbetriebs so bemessen ist, dass an wenigstens einer Stelle (46, 47, 48) zwischen der Einlassöffnung und der Auslassöffnung der Flächenmittelwert des Axialgeschwindigkeitsprofils gemittelt über den Strömungsquerschnitt wenigstens einmal vorübergehend sein Vorzeichen wechselt.

3. Pumpensystem nach Anspruch 1 oder 2, **dadurch gekennzeichnet, dass** während des Sonderbetriebs an einer Einlassöffnung und/oder einer Auslassöffnung der Gesamtvolumenstrom seine Richtung wenigstens einmal zeitweise umkehrt.

4. Pumpensystem nach Anspruch 1, **dadurch gekennzeichnet, dass** während eines Sonderbetriebs an mehreren Stellen (46, 47, 48, 50, 52), insbesondere an allen Stellen des Strömungswegs durch die Pumpe der Gesamtvolumenstrom wenigstens einmal zeitweise umgekehrt ist.

5. Pumpensystem nach Anspruch 1 oder einem der folgenden, **dadurch gekennzeichnet, dass** das Zeitintegral des Förderstroms über eine gesamte Sonderbetriebsphase negativ ist.

6. Pumpensystem mit einer Blutpumpe (5), die ein von Blut durchströmbares Gehäuse (7) und einen Antrieb mit einem Förderelement (19, 26, 27) aufweist, sowie mit einer Steuerungseinrichtung (10) zur Steuerung der Blutpumpe, wobei das Förderelement der Pumpe als Rotor (19, 26, 27) ausgebildet ist, **dadurch gekennzeichnet, dass** in der Steuerungseinrichtung wenigstens ein erstes Drehzahlniveau des Rotors für den Förderbetrieb und wenigstens ein Sonderbetriebsdrehzahlniveau (υ₂) für einen Sonderbetrieb vorgesehen ist, wobei das wenigstens eine Sonderbetriebsdrehzahlniveau eine niedrigere Drehzahl des Rotors vorsieht als der Förderbetrieb.

7. Pumpensystem nach Anspruch 6, **dadurch gekennzeichnet, dass** das wenigstens eine Sonderbetriebsdrehzahlniveau um wenigstens 25%, insbesondere 40%, weiter insbesondere 50% oder 60%, unterhalb der Drehzahl des Förderbetriebs liegt.

8. Pumpensystem nach Anspruch 6 oder 7, **dadurch gekennzeichnet, dass** über eine Zeitspanne während einer Sonderbetriebsphase ein konstantes Sonderbetriebsdrehzahlniveau, insbesondere das niedrigste Sonderbetriebsdrehzahlniveau vorgesehen ist.

9. Pumpensystem mit einer Blutpumpe (5), die ein von Blut durchströmbares Gehäuse (7) und einen Antrieb mit einem Förderelement (19, 26, 27) aufweist, sowie mit einer Steuerungseinrichtung (10) zur Steuerung der Blutpumpe, wobei das Förderelement der Pumpe als Rotor ausgebildet ist, wobei die Steuerungseinrichtung ein erstes Drehzahlniveau des Rotors für den Förderbetrieb und wenigstens ein Sonderbetriebsdrehzahlniveau (υ₂) für einen Sonderbetrieb vorsieht, **dadurch gekennzeichnet, dass** die Drehzahl während eines Sonderbetriebs wenigstens zeitweise wenigstens 2000 U/min, insbesondere 2500 U/min, weiter insbesondere 3000 U/min oder 4000 U/min unterhalb der Drehzahl im Normalbetrieb liegt.

10. Verfahren zum Betrieb einer Blutpumpe (5), die in den Blutkreislauf eines Patienten eingeführt ist, **dadurch gekennzeichnet, dass** die Pumpe abwechselnd im Förderbetrieb und im Sonderbetrieb angesteuert wird, wobei der Sonderbetrieb wenigstens zeitweise eine Umkehrung des Gesamtvolumenstroms an wenigstens einer Stelle (46, 47, 48, 50, 52) entlang des Strömungsweges zwischen einer Einlassöffnung der Pumpe und der nächsten Querschnittsverengung des Strömungsweges hinter der Auslassöffnung der Pumpe insbesondere zwischen der Einlassöffnung (50) der Pumpe und der Auslassöffnung (52) der Pumpe bewirkt.

11. Verfahren nach Anspruch 10, **dadurch gekennzeichnet, dass** eine Sonderbetriebsphase zwischen 0,5 Sekunden und 1,5 Sekunden beträgt.

12. Verfahren nach Anspruch 10 oder 11, **dadurch gekennzeichnet, dass** zwischen 0,1 und 2 Sonderbetriebsphasen pro Minute, insbesondere zwischen 1 und 2 Sonderbetriebsphasen pro Minute, vorgesehen werden.

13. Verfahren nach Anspruch 10, 11 oder 12, **dadurch gekennzeichnet, dass** während einer Sonderbetriebsphase die Drehzahl der Pumpe bis zu einer Sonderbetriebsdrehzahl (υ₂) abgesenkt und danach wieder angehoben wird.

14. Verfahren nach Anspruch 13, **dadurch gekennzeichnet, dass** die Sonderbetriebsdrehzahl wenigstens 25%, insbesondere wenigstens 30%, weiter insbesondere wenigstens 40%, 50% oder 60%, insbesondere wenigstens 2000 U/min oder 3000 U/min, unterhalb der Drehzahl im Förderbetrieb liegt.
